# EUROPÄISCHE PATENTSCHRIFT

(11) **EP 3 188 770 B1**
(45) Veröffentlichungstag und Bekanntmachung des Hinweises auf die Patenterteilung: **30.10.2019**
(21) Anmeldenummer: 15763834.7
(22) Anmeldetag: 03.09.2015
(51) Int. Cl.: A61M 1/28

(54) **VORRICHTUNG ZUR DURCHFÜHRUNG EINER TIDAL-PERITONEALDIALYSEBEHANDLUNG**
APPARATUS FOR CARRYING OUT A TIDAL PERITONEAL DIALYSIS TREATMENT
DISPOSITIF DE TRAITEMENT PAR DIALYSE PÉRITONÉALE À FLUX ET REFLUX

(30) Priorität: 05.09.2014 DE 102014013229
(43) Veröffentlichungstag der Anmeldung: 12.07.2017
(60) Teilanmeldung: 19189169.6
(73) Patentinhaber: Fresenius Medical Care Deutschland GmbH, 61352 Bad Homburg (DE)
(72) Erfinder: WOLF, Klaus, 97450 Arnstein-Müdesheim (DE); GRIESSMANN, Erik, 97422 Schweinfurt (DE)
(74) Vertreter: Herrmann, Uwe
(86) Internationale Anmeldenummer: PCT/EP2015/001783
(87) Internationale Veröffentlichungsnummer: WO 2016/034287

(56) Entgegenhaltungen:
- EP-A1- 1 691 863
- WO-A2-2012/036836
- US-A1- 2013 165 847

## Beschreibung

Die vorliegende Erfindung betrifft eine Vorrichtung zur Durchführung einer Tidal-Peritonealdialysebehandlung eines Patienten, wobei die Dialysebehandlung eine Abfolge von Zyklen umfasst, die jeweils eine Füllperiode, eine Verweilperiode und eine Ablaufperiode aufweisen, wobei die Vorrichtung wenigstens einen Cycler aufweist, der zur Füllung der Bauchhöhle des Patienten mit einem bestimmten Einlaufvolumen an frischer Dialyselösung bis zum Erreichen eines bestimmten Patientenvolumens in der Füllperiode und zum Ablassen verbrauchter Dialyselösung aus der Bauchhöhle des Patienten bis zum Erreichen eines bestimmten Tidal-Auslaufziels in der Ablaufperiode geeignet ist.

Im Rahmen der Peritonealdialyse wird eine Dialyselösung während einer Füllperiode in die Bauchhöhle des zu behandelnden Patienten eingeleitet. Diese verbleibt dort für eine Verweilperiode, in der es zu einem Stoffaustausch mit dem Blut des Patienten kommt, wodurch das Blut gereinigt wird. Nach Ablauf der Verweilperiode wird die Dialyselösung während der Ablaufperiode wieder abgelassen.

Diese Vorgehensweise wird über eine Mehrzahl von aufeinander folgenden Zyklen wiederholt. Dazu dient der sogenannte Cycler, der eine oder mehrere Pumpen umfasst, die das sukzessive Füllen und Ablassen von Dialyselösung in die bzw. aus der Bauchhöhle durchführen.

Aus dem Stand der Technik sind Peritonealdialyseverfahren bekannt, bei denen die Dialyselösung während der Ablaufperiode vollständig abgelassen wird. Des Weiteren ist die so genannte Tidal-Peritonealdialysebehandlung bekannt, bei der ein anfängliches Füllvolumen eingefüllt wird, bei den Ablaufperioden aber jeweils nur ein Teil dieses Füllvolumens abgelassen wird, d.h. es verbleibt stets ein gewisses Volumen an Dialyselösung im Patienten bis die Behandlung vollständig abgeschlossen ist. Der Cycler wird somit derart betrieben, dass die Ablaufperiode bis zum Erreichen eines bestimmten Tidal-Auslaufziels erfolgt, d.h. solange bis ein bestimmtes Volumen an Dialyselösung aus der Bauchhöhle abgezogen wurde. Dieses bestimmte abgezogene Volumen an verbrauchter Dialyselösung wird im Rahmen der vorliegenden Erfindung als Tidal-Auslaufziel bezeichnet.

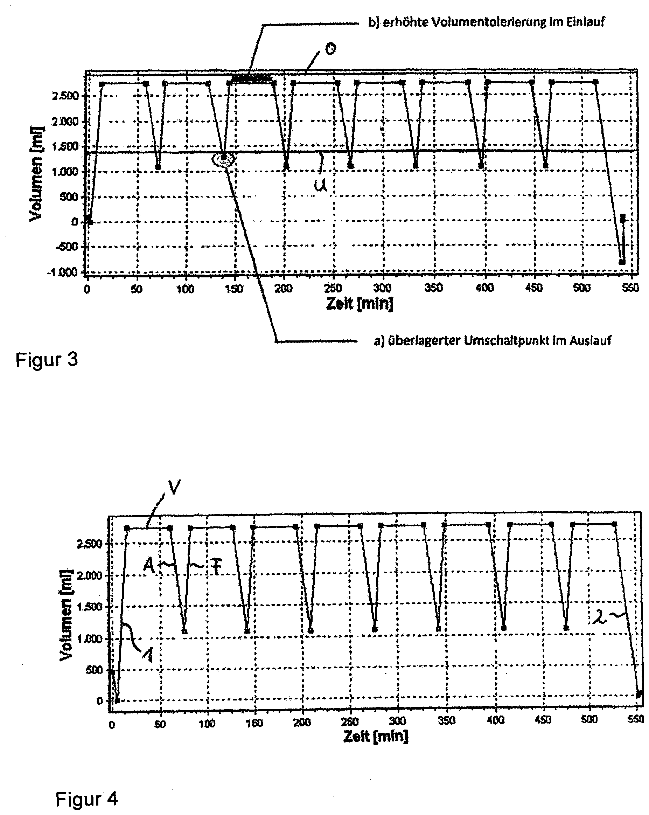

Die zeitliche Abfolge der Schritte einer bekannten Tidal-Peritonealdialysebehandlung zeigt Figur 4. Die Ordinate zeigt das in dem Patienten befindliche Volumen an Dialyselösung, die Abszisse zeigt die Zeit.

Zu Beginn erfolgt in Schritt 1 die Füllung der Bauchhöhle mit einem Volumen an Dialyselösung. Nach Ablauf einer bestimmten Verweilperiode V wird während der Ablaufperiode A bis zum Erreichen eines bestimmten Tidal-Auslaufziels verbrauchte Dialyselösung aus der Bauchhöhle abgelassen. Wie dies aus Figur 4 hervorgeht, werden im Rahmen der Ablaufperiode A in dem hier dargestellten Beispiel ca. 1600 ml (2700 ml - 1100 ml) abgelassen. Das Tidal-Auslaufziel beträgt in dem Beispiel somit 1600 ml. Im Anschluss an die Ablaufperiode A erfolgt eine erneute Füllung der Bauchhöhle mit einem bestimmten Einlaufvolumen (hier ebenfalls 1600 ml) während der Füllperiode F bis ein bestimmtes Patientenvolumen, d.h. ein bestimmtes Volumen von Dialyselösung in der Bauchhöhle erreicht ist. In dem dargestellten Beispiel beträgt dieses Volumen 2700 ml.

Nach Durchführung einer Anzahl von Zyklen wird die Dialyselösung aus der Bauchhöhle vollständig abgelassen (Schritt 2).

Bei der Tidal-Verschreibung ist das Austauschvolumen, d.h. das im Rahmen der Ablaufperiode aus dem Patienten abgeführte Volumen an Dialyselösung vorgegeben (volumengesteuerter Auslauf). Es wird somit ein fest vorgegebenes Volumen in den Ablauf entleert. Wie dies weiter aus Figur 4 hervorgeht, ist dieses Volumen im Vergleich zu dem im Basis-Zyklus (Schritte 1 und 2) ausgetauschten Volumen verringert. Dies wird in vielen Anwendungsfällen aus Gründen der Alarmminimierung oder der Alarmvermeidung während der im Allgemeinen nächtlichen APD-Behandlung (APD = Automatisierte Peritonealdialyse) angewandt.

Die Reduzierung der Austauschvolumina der einzelnen Tidal-Zyklen bewirkt eine Reduzierung der Austauschmengen der Dialyselösung Um dennoch während einer bestimmten Behandlungszeit ein vorgegebenes Volumen an Dialyselösung verabreichen zu können, werden die einzelnen Verweilperioden V kurz gehalten und die Anzahl der Zyklen wird entsprechend groß gewählt. Bei der bekannten Tidal-Peritonealdialysebehandlung kommt es somit zu einem Kompromiss zwischen kurzen Verweilzeiten, niedrigen Auslaufmengen und verabreichter Dialyselösung. Dieser Kompromiss wirkt sich unmittelbar auf die Dialyseeffektivität aus.

Folgende sind relevante Stände der Technik: EP 1 691 863 A1, WO 2012/036836 A2, US 2013/165847 A1. Aus der EP 1 691 863 B1 ist eine Peritonealdialysebehandlung bekannt, bei der während der Ablaufperiode ein Parameter erfasst wird, der für die Ablaufrate repräsentativ ist. Bei Erfassung einer wesentlichen Änderung des Wertes dieses Parameters wird die Ablaufperiode beendet und auf eine Füllperiode umgeschaltet. Die beschriebene Vorgehensweise kann dazu führen, dass am Ende der Behandlung, d.h. bei Ablauf der Behandlungsdauer noch ein Restvolumen an frischer Dialyselösung übrig ist, so dass ein weiterer Zyklus an die Behandlung angefügt werden muss, um auch diese Lösung verwenden zu können. Weiterhin geht diese Vorgehensweise in der Regel mit einer Verkürzung der Verweilzeit der Dialyselösung in der Bauchhöhle einher.

Kann das Tidal-Auslaufziel nicht erreicht werden, wird entsprechend auch das Einlaufvolumen des folgenden Zyklus reduziert, um ein Aufkumulieren von Volumen im Patienten zu vermeiden. Das Reduzieren des Einlaufvolumens führt bei vorgegebener Behandlungsdauer zu einer nicht vollständigen Verwertung der Dialyselösung, d.h. es kann nicht die gesamte vorgesehene Dialyselösung verabreicht werden.

Der vorliegenden Erfindung liegt die Aufgabe zugrunde, eine Vorrichtung der eingangs genannten Art dahingehend weiterzubilden, dass im Rahmen der Behandlung ein großes Austauschvolumen, d.h. ein großes Volumen der dem Patienten zugeführten Dialyselösung verabreicht werden kann.

Diese Aufgabe wird durch eine Vorrichtung mit den Merkmalen des Anspruchs 1 gelöst.

Danach ist vorgesehen, dass die als Peritonealdialysegerät ausgebildete Vorrichtung des Weiteren wenigstens einen Prozessor aufweist, der den Cycler in wenigstens einem Betriebsmodus derart ansteuert, dass die Ablaufperiode vor Erreichen des Tidal-Auslaufziels beendet wird und sodann in eine Füllperiode umgeschaltet wird, so dass ein zulässiges Residualvolumen in der Bauchhöhle verbleibt, und dass die Füllperiode derart durchgeführt wird, dass das Füllvolumen des Patienten bei Beendigung der Füllperiode bei einem zulässigen Patientenvolumen oberhalb des bestimmten Patientenvolumens liegt.

Das Tidal-Auslaufziel und/oder das bestimmte Einlaufvolumen und/oder das bestimmte Patientenvolumen sind fest vorgegebene Werte oder nutzerseitig veränderbare Werte. Diese sind vorzugsweise in einem oder in mehreren Speichern des Gerätes abgelegt.

Das erfindungsgemäße Volumenmanagement sieht somit vor, dass die Ablaufperiode beendet wird, wenn eine Auslaufvolumengrenze oder ein Auslaufvolumenbereich erreicht ist. Diese/dieser liegt oberhalb des Tidal-Auslaufziels. Somit wird in wenigstens einem Betriebsmodus der Vorrichtung nicht solange entleert, bis das vorgeschriebene Tidal-Auslaufziel eines Zyklus erreicht ist, sondern bereits vorher die Ablaufperiode abgebrochen und in eine neue Füllperiode übergegangen.

Des Weiteren wird in einer darauf folgenden Füllperiode ein gegenüber dem bestimmten Patientenvolumen erhöhtes maximales Patientenvolumen, d.h. ein erhöhtes maximales Volumen von in dem Patienten befindlicher Dialyselösung toleriert. Das zulässige Patientenvolumen liegt somit über dem bestimmten Patientenvolumen einer üblichen Tidal-Peritonealdialysebehandlung. Bezogen auf das eingangs genannte Beispiel liegt das bestimmte Patientenvolumen bei 2700 ml und das zulässige Patientenvolumen beispielsweise bei 3000 ml.

Auf diese Weise lassen sich sowohl hohe Austauschmengen (Tidal-Auslaufvolumina) als auch das Ziel der Alarmvermeidung oder -verringerung erreichen.

Vorzugsweise ist vorgesehen, dass das zulässige Residualvolumen und/oder das zulässige Patientenvolumen einstellbar ist. Da in wenigstens einem Betriebsmodus erfindungsgemäß die Ablaufperiode vor Erreichen des Tidal-Auslaufziels abgebrochen wird, ergibt sich ein erhöhtes Volumen von in der Bauchhöhle verbleibender Dialyselösung; dieses Volumen wird im Rahmen der Erfindung als Residualvolumen bezeichnet.

Besonders vorteilhaft ist es, wenn das zulässige Residualvolumen und das zulässige Patientenvolumen unabhängig voneinander einstellbar sind. Eine unabhängige und individuelle Einstellbarkeit dieser Werte ermöglicht eine optimale Anpassung der Behandlung an den Patienten.

Vorzugsweise ist vorgesehen, dass das erfindungsgemäße Abbrechen der Ablaufperiode und das anschließende Füllen bis zu einem zulässigen Patientenvolumen, d.h. das erfindungsgemäße Volumenmanagement nur bei Bedarf angewandt wird. Dies kann der Fall sein, wenn festgestellt wird, dass das Tidal-Auslaufziel nicht oder nicht in einer bestimmten Zeitspanne ab Beginn der Ablaufperiode erreicht wird.

Denkbar ist es somit, dass der Prozessor derart ausgebildet ist, dass die Wahl des Betriebsmodus von dem Erreichen des Tidal-Auslaufziels abhängt. Wird festgestellt, dass das Tidal-Auslaufziel nicht oder nicht innerhalb einer bestimmten Zeitspanne erreicht wird, kann der erfindungsgemäße Betriebsmodus gewählt werden, in dem die Ablaufperiode vor dem Erreichen des Tidal-Auslaufziels abgebrochen wird und in der Füllperiode das Füllen bis zu einem zulässigen Patientenvolumen oberhalb des bestimmten Patientenvolumens erfolgt. Wird jedoch festgestellt, dass das Tidal-Auslaufziel erreicht wird, kann die Vorrichtung ohne diese Maßnahmen, d.h. herkömmlich wie in Figur 4 dargestellt durchgeführt werden. In diesem Fall erfolgt das Umschalten von der Ablaufperiode zur Füllperiode nicht vor, sondern bei dem Erreichen des Tidal-Auslaufvolumens und dass Füllen in der Füllperiode nicht oberhalb, sondern bei Erreichen des bestimmten Patientenvolumens.

In einer Ausgestaltung der Erfindung ist vorgesehen, dass der Prozessor derart ausgebildet ist, dass der Zeitpunkt, zu dem die Ablaufperiode beendet wird, von wenigstens einem Parameter abhängt. Bei diesem Parameter, zu dessen Messung wenigstens ein Sensor vorhanden sein kann, kann es sich um das erreichte Ablaufvolumen und/oder die Flussrate der Dialyselösung während der Ablaufperiode sowie um den Druck in der Bauchhöhle des Patienten handeln.

Der Prozessor ist derart ausgebildet, dass der Umschaltwert, bei dem die Ablaufperiode beendet wird, einen bestimmten Prozentsatz des Tidal-Auslaufziels, d.h. des während einer Ablaufperiode gemäß der Verschreibung abzuführenden Volumens an Dialyselösung oder des bestimmten Einlaufvolumens beträgt, wobei vorzugweise vorgesehen ist, dass der Prozentsatz im Bereich von 0 bis 60 % und vorzugweise im Bereich von 10 bis 50 % des Tidal-Auslaufziels bzw. des bestimmten Einlaufvolumens liegt. Nimmt man den Wert von 10 % an und beträgt das Tidal-Auslaufziel 1 l, nimmt der Grenzwert den Wert 100 ml an. Dies bedeutet, dass bei einem Auslaufvolumen von 900 ml (1 l - 100 ml), d.h. 100 ml vor dem Erreichen des Tidal-Auslaufziels umgeschaltet wird.

Für den unteren Umschaltpunkt, zu dem die Ablaufperiode abgebrochen wird, kann somit beispielsweise ein Bereich von 0 bis 60 % und vorzugweise im Bereich von 10 bis 50 % des höchsten Einlaufvolumens vorgesehen sein. Bei einem Einlaufvolumen oder bei einem Tidal-Auslaufziel von 1 I kann daher der Umschaltpunkt in einem Bereich von 100 bis 500 ml des vorgesehenen Drainvolumens liegen. Somit kann umgeschaltet werden, d.h. die Ablaufperiode beendet werden, wenn während der Ablaufperiode 500 ml bis 900 ml an verbrauchter Dialyselösung abgezogen wurden.

Des Weiteren kann der Prozessor derart ausgebildet sein, dass das zulässige Patientenvolumen oder das im Rahmen der Füllperiode zugegebene Volumen im Bereich von über 100 bis 150 %, vorzugsweise im Bereich von über 100 bis 120 % und besonders bevorzugt im Bereich von über 100 bis 130 % des bestimmten Patientenvolumens oder des bestimmten Einlaufvolumens liegt. Für den oberen Bereich, d.h. für den oberen Umschaltpunkt wird max. zulässiges Volumen von über 100 % (100 % = kein zusätzliches Patientenvolumen wird toleriert) bis 150 % toleriert. D.h. bei einem verschriebenen Einlaufvolumen von 1 I darf in dieser Ausgestaltung der Erfindung weitere Dialyselösung bis zu einem kumulierten Einlaufvolumen von max. 1,5 I zugegeben werden.

Diese Offenbarung betrifft des Weiteren ein Verfahren zur Durchführung einer Tidal-Peritonealdialysebehandlung, die eine Abfolge von Zyklen umfasst, die jeweils eine Füllperiode, eines Verweilperiode und eine Ablaufperiode aufweisen, wobei im Normalbetrieb während der Füllperiode die Füllung der Bauchhöhle des Patienten mit einem bestimmten Einlaufvolumen an frischer Dialyselösung bis zum Erreichen eines bestimmten Patientenvolumens und in der Ablaufperiode das Ablassen verbrauchter Dialyselösung aus der Bauchhöhle des Patienten bis zum Erreichen eines bestimmten Tidal-Auslaufziels erfolgt, wobei in wenigstens einem vom Normalbetrieb abweichenden Betriebsmodus die Ablaufperiode vor Erreichen des Tidal-Auslaufziels beendet wird, so dass ein zulässiges Residualvolumen in der Bauchhöhle verbleibt, und sodann in eine Füllperiode umgeschaltet wird und dass die Füllperiode derart durchgeführt wird, dass das Füllvolumen des Patienten mit Dialyselösung bei Beendigung der Füllperiode bei einem zulässigen Patientenvolumen oberhalb des bestimmten Patientenvolumens liegt.

Vorzugsweise ist vorgesehen, dass das zulässige Residualvolumen und/oder das zulässige Patientenvolumen einstellbar ist. Vorteilhaft ist es, wenn das zulässige Residualvolumen und das zulässige Patientenvolumen unabhängig voneinander einstellbar sind. Dies kann für einen oder beide der Umschaltpunkte (unterer Umschaltpunkt zwischen der Ablaufperiode zur Füllperiode und/oder oberer Umschaltpunkt von der Füllperiode zur Verweilperiode) entsprechend gelten.

In einer weiteren Ausgestaltung ist die Wahl des Betriebsmodus von dem Erreichen des Tidal-Auslaufziels abhängig. Wird das Tidal-Auslaufziel erreicht bzw. innerhalb einer bestimmten Zeitspanne erreicht, kann auf das Abrechen des Ablaufperiode sowie auf einer Erhöhung des dem Patienten verabreichten Volumens über das bestimmte Patientenvolumen hinaus verzichtet werden. Ist dies jedoch nicht der Fall, wird das offenbarte Verfahren angewandt.

Der Zeitpunkt, zu dem die Ablaufperiode beendet wird, kann von wenigstens einem Parameter abhängen, wobei es sich bei dem Parameter um eine oder mehrere der folgenden Größen handelt kann: erreichtes Ablaufvolumen, Flussrate während der Ablaufperiode sowie Druck in der Bauchhöhle des Patienten.

Der Umschaltwert, bei dem die Ablaufperiode beendet wird, kann einen bestimmten Prozentsatz des Tidal-Auslaufziels oder des bestimmten Einlaufvolumens betragen, wobei vorzugweise vorgesehen ist, dass der Prozentsatz im Bereich von über 0 bis 60 % und vorzugweise im Bereich von 10 bis 50 % des Tidal-Auslaufziels bzw. des bestimmten Einlaufvolumens liegt.

Weiterhin kann vorgesehen sein, dass das zulässige Patientenvolumen oder das während einer Füllperiode zugegebene Volumen im Bereich von über 100 bis 150 %, vorzugsweise im Bereich von über 100 bis 120 % und besonders bevorzugt im Bereich von über 100 bis 130 % des bestimmten Patientenvolumens bzw. des bestimmten Einlaufvolumens liegt.

Weitere Einzelheiten und Vorteile der Erfindung werden anhand eines in der Zeichnung dargestellten Ausführungsbeispiels näher erläutert.

Es zeigen:
- Figur 1:: zeitlicher Ablauf einer Tidal-Peritonealdialysebehandlung mit unterem Umschaltpunkt,
- Figur 2:: zeitlicher Ablauf einer Tidal-Peritonealdialysebehandlung mit oberem und mit unterem Umschaltpunkt,
- Figur 3:: zeitlicher Ablauf einer Tidal-Peritonealdialysebehandlung mit unterem Umschaltpunkt mit Kennzeichnung des überlagerten Umschaltpunktes im Auslauf und erhöhter Volumentolerierung im Einlauf und
- Figur 4:: zeitlicher Ablauf einer Tidal-Peritonealdialysebehandlung gemäß dem Stand der Technik.

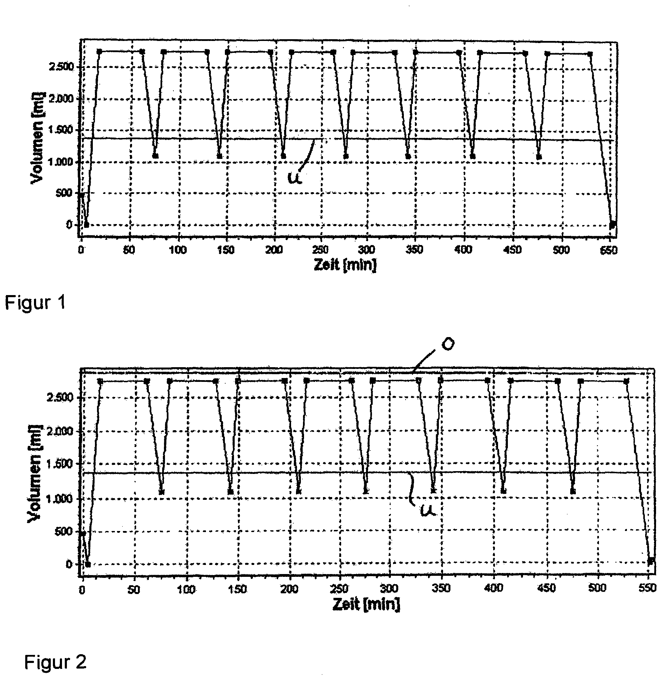

Figur 1 zeigt den zeitlichen Verlauf einer Tidal-Peritonealdialysebehandlung gemäß Figur 4 mit überlagerter unterer Volumenmanagementgrenze im Form der Linie U. Diese Linie stellt den Umschaltpunkt bzw. den Volumenwert dar, bei dessen Unterschreiten in der Ablaufperiode gemäß dem erfindungsgemäßen Betriebsmodus ein Umschalten auf die nächste Füllperiode erfolgt. Wie dies aus Figur 1 ersichtlich ist, liegt diese Linie über den Umschaltpunkten bei einer Vorgehensweise gemäß dem Stand der Technik. Letztere liegen in dem hier gezeigten Ausführungsbeispiel bei einem Volumen von 1100 ml. Die Linie U bzw. die Umschaltpunkte gemäß der Erfindung liegen bei einem Volumen von 1400 ml bzw. in einem Volumenbereich von > 1100 ml bis 1400 ml. Bei den angegebenen Volumenwerten handelt es sich um das Volumen der Dialyselösung in der Bauchhöhle des Patienten. Das bei der Linie U im Patienten befindliche Volumen der Dialyselösung wird im Rahmen der Erfindung als zulässiges Residualvolumen bezeichnet.

Figur 2 zeigt den zeitlichen Verlauf einer Tidal-Peritonealdialysebehandlung gemäß Figur 1 mit überlagerter oberer Volumenmanagementgrenze im Form der Linie O. Diese Linie stellt den Umschaltpunkt bzw. den Volumenwert dar, bei dessen Erreichen oder Überschreiten eine Beendigung der Füllperiode und ein Beginn der Verweilperiode erfolgt. Wie dies aus Figur 2 ersichtlich ist, liegt diese Linie O über den oberen Umschaltpunkten (Umschalten auf die Verweilperiode) bei einer Vorgehensweise gemäß dem Stand der Technik. Letztere liegen in dem hier gezeigten Ausführungsbeispiel bei einem Volumen von 2700 ml. Die Linie O bzw. die Umschaltpunkte gemäß der Erfindung liegen bei einem Volumen von 2900 ml bzw. in einem Volumenbereich von > 2700 ml bis 2900 ml. Bei den angegebenen Volumenwerten handelt es sich um das Volumen der Dialyselösung in der Bauchhöhle des Patienten. Das bei der Linie O im Patienten befindliche Volumen der Dialyselösung wird im Rahmen der Erfindung als zulässiges Patientenvolumen bezeichnet. Figur 2 verdeutlicht somit die zusätzliche Volumentolerierung im Einlauf, d.h. während der Füllperiode.

Figur 3 verdeutlicht, dass das Tidal-Auslaufziel (Volumen 1100 ml) im zweiten Zyklus nicht bzw. jedenfalls nicht innerhalb einer bestimmten Zeitspanne erreicht wird, weil die Auslaufgeschwindigkeit vergleichsweise gering ist, wie dies an der geringeren Steigung der Linie erkennbar ist.

Dies führt dazu, dass der Ablauf zu dem mit a) gekennzeichneten Zeitpunkt abgebrochen wird bevor das Tidal-Auslaufziel erreicht ist und auf eine neue Füllperiode umgeschaltet wird.

Es wird somit ein erhöhtes Residualvolumen in Kauf genommen. Um dennoch ein hinreichendes Volumen an Dialyselösung verabreichen zu können, wird in der folgenden Füllperiode eine Überschreitung des bestimmten Patientenvolumens in Höhe von 2700 ml hingenommen und die Füllperiode wird verlängert, bis das Patientenvolumen, d.h. das in dem Patienten befindliche Volumen an Dialyselösung 2900 ml beträgt.

Würde in dieser Füllperiode nur bis zu dem bestimmten Patientenvolumen in Höhe von 2700 ml aufgefüllt, ließe sich in der vorbestimmten Behandlungszeit insgesamt nur ein geringeres Volumen an Dialyselösung verabreichen bzw. es wären bei Überschreitung der vorbestimmten Behandlungszeit ein oder mehrere weitere Zyklen notwendig.

Mit dem erfindungsgemäßen Volumenmanagement ist es möglich, bereits in der Verschreibung ein höheres Auslaufvolumen festzulegen und somit eine effizientere Nutzung der Dialyselösung zu erreichen.

Die Notwendigkeit, einen oder mehrere zusätzliche Zyklen durchzuführen kann bei der erfindungsgemäßen Vorgehensweise entfallen. Des Weiteren kann eine Verkürzung der Verweilzeiten vermieden werden und der Patient kann gleichwohl zum vorgesehenen Zeitpunkt die Behandlung beenden.

## Patentansprüche

1. Vorrichtung zur Durchführung einer Tidal-Peritonealdialysebehandlung eines Patienten, wobei die Dialysebehandlung eine Abfolge von Zyklen umfasst, die jeweils eine Füllperiode, eines Verweilperiode und eine Ablaufperiode aufweisen, wobei die Vorrichtung wenigstens einen Cycler aufweist, der zur Füllung der Bauchhöhle des Patienten mit einem bestimmten Einlaufvolumen an frischer Dialyselösung bis zum Erreichen eines bestimmten Patientenvolumens in der Füllperiode und zum Ablassen verbrauchter Dialyselösung aus der Bauchhöhle des Patienten bis zum Erreichen eines bestimmten Tidal-Auslaufziels in der Ablaufperiode geeignet ist, **dadurch gekennzeichnet, dass** die Vorrichtung des Weiteren wenigstens einen Prozessor aufweist, der den Cycler in wenigstens einem Betriebsmodus derart ansteuert, dass die Ablaufperiode vor Erreichen des Tidal-Auslaufziels beendet wird, so dass ein zulässiges Residualvolumen in der Bauchhöhle verbleibt, und sodann in eine Füllperiode umgeschaltet wird und dass die Füllperiode derart durchgeführt wird, dass das Füllvolumen des Patienten bei Beendigung der Füllperiode bei einem zulässigen Patientenvolumen oberhalb des bestimmten Patientenvolumens liegt, wobei der Prozessor derart ausgebildet ist, dass der Umschaltwert, bei dem die Ablaufperiode beendet wird, einen bestimmten Prozentsatz des Tidal-Auslaufziels oder des bestimmten Einlaufvolumens beträgt, wobei vorzugweise vorgesehen ist, dass der Prozentsatz im Bereich von über 0 bis 60 % und vorzugweise im Bereich von 10 bis 50 % des Tidal-Auslaufziels oder des bestimmten Einlaufvolumens liegt.

2. Vorrichtung nach Anspruch 1, **dadurch gekennzeichnet, dass** das zulässige Residualvolumen und/oder das zulässige Patientenvolumen einstellbar ist.

3. Vorrichtung nach Anspruch 1 oder 2, **dadurch gekennzeichnet, dass** das zulässige Residualvolumen und das zulässige Patientenvolumen unabhängig voneinander einstellbar sind.

4. Vorrichtung nach einem der vorhergehenden Ansprüche, **dadurch gekennzeichnet, dass** der Prozessor derart ausgebildet ist, dass die Wahl des Betriebsmodus von dem Erreichen des Tidal-Auslaufziels abhängt.

5. Vorrichtung nach einem der vorhergehenden Ansprüche, **dadurch gekennzeichnet, dass** der Prozessor derart ausgebildet ist, dass der Zeitpunkt, zu dem die Ablaufperiode beendet wird, von wenigstens einem Parameter abhängt, wobei es sich bei dem Parameter um eine oder mehrere der Größen erreichtes Ablaufvolumen, Flussrate während der Ablaufperiode sowie Druck in der Bauchhöhle des Patienten handelt.

6. Vorrichtung nach einem der vorhergehenden Ansprüche, **dadurch gekennzeichnet, dass** der Prozessor derart ausgebildet ist, dass das zulässige Patientenvolumen oder das während einer Füllperiode zugegebene Volumen im Bereich von über 100 bis 150 %, vorzugsweise im Bereich von über 100 bis 120 % und besonders bevorzugt im Bereich von über 100 bis 130 % des bestimmten Patientenvolumens bzw. des bestimmten Einlaufvolumens liegt.

## Claims

1. An apparatus for carrying out a tidal peritoneal dialysis treatment of a patient, wherein the dialysis treatment comprises a sequence of cycles which each have a filling period, a dwell period and a drain period, wherein the apparatus has at least one cycler which is suitable for filling the abdominal cavity of the patient with a specific inflow volume of fresh dialysis solution up to a reaching of a specific patient volume in the filling period and for emptying consumed dialysis solution from the abdominal cavity of the patient up to a reaching of a specific tidal outflow target in the drain period **characterized in that** the apparatus furthermore has at least one processor which controls the cycler in at least one operating mode such that the drain period is ended before reaching the tidal outflow target so that a permitted residual volume remains in the abdominal cavity and then a switch is made to a filling period and **in that** the filling period is carried out such that the filling volume of the patient lies at a permitted patient volume above the specific patient volume on the completion of the filling period, wherein the processor is configured such that the switchover value at which the drain period is ended amounts to a specific percentage of the tidal outflow target or of the specific inflow volume, with provision preferably being made that the percentage lies in the range from above 0 to 60%, and preferably in the range from 10 to 50%, of the tidal outflow target or of the specific inflow volume.

2. An apparatus in accordance with claim 1, **characterized in that** the permitted residual volume and/or the permitted patient volume can be adjusted.

3. An apparatus in accordance with claim 1 or claim 2, **characterized in that** the permitted residual volume and the permitted patient volume can be adjusted independently of one another.

4. An apparatus in accordance with one of the preceding claims, **characterized in that** the processor is configured such that the selection of the operating mode depends on the reaching of the tidal outflow target.

5. An apparatus in accordance with one of the preceding claims, **characterized in that** the processor is configured such that the time at which the drain period is ended depends on at least one parameter, with this parameter being one or more of the values achieved volume; flow rate during the drain period; and pressure in the abdominal cavity of the patient.

6. An apparatus in accordance with one of the preceding claims, **characterized in that** the processor is configured such that the permitted patient volume or the volume added within the course of a filling period lies in the range from above 100 to 150%, preferably in the range from above 100 to 120%, and particularly preferably in the range from above 100 to 130%, of the specific patient volume or of the specific inflow volume.

## Revendications

1. Dispositif servant à mettre en œuvre un traitement par dialyse péritonéale à flux et reflux d'un patient, dans lequel le traitement par dialyse comprend une succession de cycles, qui présentent respectivement une période de remplissage, une période de séjour et une période d'évacuation, dans lequel le dispositif présente au moins un cycleur, qui est adapté pour remplir la cavité abdominale du patient avec un volume d'apport défini en solution de dialyse fraîche jusqu'à atteindre un volume de patient défini dans la période de remplissage et pour évacuer la solution de dialyse usagée hors de la cavité abdominale du patient jusqu'à atteindre un objectif d'évacuation par flux et reflux défini dans la période d'évacuation, **caractérisé en ce que** le dispositif présente par ailleurs au moins un processeur, qui pilote le cycleur dans au moins un mode de fonctionnement de telle manière que la période d'évacuation est terminée avant d'atteindre l'objet d'évacuation par flux et reflux de sorte qu'un volume résiduel admis reste dans la cavité abdominale et puis une commutation sur une période de remplissage est réalisée, et que la période de remplissage est mise en œuvre de telle manière que le volume de remplissage du patient à la fin de la période de remplissage est supérieur au volume de patient défini pour un volume de patient admis, dans lequel le processeur est configuré de telle manière que la valeur de commutation, à laquelle la période d'évacuation est finie, est égale à un pourcentage défini de l'objectif d'évacuation par flux et reflux ou du volume d'apport défini, dans lequel il est prévu de préférence que le pourcentage se situe dans la plage supérieure à 0 à 60 % et de préférence dans la plage de 10 à 50 % de l'objectif d'évacuation par flux et reflux ou du volume d'apport défini.

2. Dispositif selon la revendication 1, **caractérisé en ce que** le volume résiduel acceptable et/ou le volume de patient acceptable sont réglables.

3. Dispositif selon la revendication 1 ou 2, **caractérisé en ce que** le volume résiduel acceptable et le volume de patient acceptable sont réglables indépendamment l'un de l'autre.

4. Dispositif selon l'une quelconque des revendications précédentes, **caractérisé en ce que** le processeur est configuré de telle manière que le choix du mode de fonctionnement dépend de l'atteinte de l'objectif d'évacuation par flux et reflux.

5. Dispositif selon l'une quelconque des revendications précédentes, **caractérisé en ce que** le processeur est réalisé de telle manière que le moment, où la période d'évacuation est finie, dépend d'au moins un paramètre, dans lequel le paramètre est une ou plusieurs des grandeurs : volume d'évacuation atteint, débit pendant la période d'évacuation ainsi que pression dans la cavité abdominale du patient.

6. Dispositif selon l'une quelconque des revendications précédentes, **caractérisé en ce que** le processeur est configuré de telle manière que le volume de patient admis ou le volume ajouté pendant une période de remplissage se situe dans la plage supérieure à 100 à 150 %, de préférence dans la plage supérieure à 100 à 120 % et de manière particulièrement préférée dans la plage supérieure à 100 à 130 % du volume du patient défini ou du volume d'apport défini.
